(19) Europäisches Patentamt
European Patent Office
Office européen des brevets

(11) Veröffentlichungsnummer : **0 088 294**
**B1**

(12) # EUROPÄISCHE PATENTSCHRIFT

(45) Veröffentlichungstag der Patentschrift : **15.05.85**

(51) Int. Cl.[4] : **C 07 C 1/04**, B 01 J 23/78

(21) Anmeldenummer : **83101798.3**

(22) Anmeldetag : **24.02.83**

(54) **Verfahren zur Herstellung von linearen Kohlenwasserstoffen mit mehr als 18 Kohlenstoffatomen.**

(30) Priorität : **04.03.82 DE 3207743**

(43) Veröffentlichungstag der Anmeldung : **14.09.83 Patentblatt 83/37**

(45) Bekanntmachung des Hinweises auf die Patenterteilung : **15.05.85 Patentblatt 85/20**

(84) Benannte Vertragsstaaten : **DE FR GB IT NL**

(56) Entgegenhaltungen :
**DE-C- 763 233**
**GB-A- 728 074**
**US-A- 2 553 433**
**US-A- 4 261 865**

(73) Patentinhaber : **Ruhrchemie Aktiengesellschaft**
**Bruchstrasse 219**
**D-4200 Oberhausen 13 (DE)**

(72) Erfinder : **Frohning, Carl Dieter, Dr. Dipl.-Chem.**
**Elsenbruch 1**
**D-4200 Oberhausen 13 (DE)**
Erfinder : **Moraw, Klaus, Dr. Dipl.-Chem.**
**Sittermannstrasse 20**
**D-4133 Neukirchen-Vluyn (DE)**

(74) Vertreter : **Reichelt, Karl-Heinz, Dr.**
**m. Br. Ruhrchemie Aktiengesellschaft Abt. PLD Postfach 13 01 60**
**D-4200 Oberhausen 13 (DE)**

**EP 0 088 294 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von linearen Kohlenwasserstoffen mit mehr als 18 Kohlenstoffatomen aus Synthesegas, d. h. dem Gemisch von Kohlenmonoxid und Wasserstoff. Die genannten Kohlenwasserstoffe sind wachsartig und finden in der industriellen Technik verbreitet Anwendung.

Es ist bekannt, eine Reihe wertvoller organischer Verbindungen durch katalytische Druckhydrierung von Kohlenoxiden, insbesondere von Kohlenmonoxid, zu gewinnen. Für diesen Zweck werden eine Anzahl Katalysatoren verwendet, die qualitativ und quantitativ unterschiedliche Zusammensetzung aufweisen und entsprechend ihrer Zusammensetzung zu verschiedenen Produkten führen. Üblicherweise ergeben die Kohlenoxidhydrierungsverfahren als Hauptprodukte Kohlenwasserstoffe und sauerstoffhaltige organische Verbindungen, wobei die genannten Verbindungsklassen komplex aufgebaut sind und jeweils Moleküle verschiedener Größe enthalten.

Nach einem vorbeschriebenen Verfahren setzt man zur Herstellung höherer Kohlenwasserstoffe Kohlenmonoxid und Wasserstoff an einem alkalisierten Eisen-Kupfer-Kataysator bei Temperaturen oberhalb 220 °C und Drücken von 20 bis 30 bar um.

Ein anderer Prozeß zur Umwandlung von Synthesegas in $\alpha$-Olefine mit 2 bis etwa 22 Kohlenstoffatome verwendet Alkalihydroxid enthaltende Eisentitanatkatalysatoren (vgl. US-PS 42 61 865).

Die bekannten Verfahren genügen noch nicht in jeder Hinsicht den Ansprüchen, die an technische Prozesse gestellt werden. Insbesondere ihre Selektivität ist noch deutlich verbesserungsbedürftig.

Der Erfindung liegt daher die Aufgabe zugrunde, die Selektivität der Reaktion von Kohlenmonoxid und Wasserstoff zu höheren, d. h. wachsartigen Kohlenwasserstoffen zu verbessern, so daß die Molekülgröße der gebildeten Kohlenwasserstoffe möglichst einheitlich ist und daß neben Kohlenwasserstoffen keine weiteren Verbindungen, z. B. sauerstoffhaltige Produkte, gebildet werden.

Die vorstehend beschriebene Aufgabe löst die Erfindung durch ein Verfahren zur Herstellung von linearen Kohlenwasserstoffen mit mehr als 18 Kohlenstoffatomen durch Umsetzung von Kohlenmonoxid und Wasserstoff enthaltenden Gasgemischen an Eisen enthaltenden Katalysatoren bei 150 bis 350 °C und 5 bis 50 bar, dadurch, daß die Katalysatoren zusätzlich ein leichtreduzierbares Metalloxid, ein schwerreduzierbares Metalloxid, $SiO_2$ und Alkalimetalloxid enthalten.

Ein wesentliches Merkmal der vorliegenden Erfindung ist der Einsatz eines Katalysators, der neben Eisen zwei Metalloxide unterschiedlicher Reduzierbarkeit enthält.

Katalysatoren dieser Art sind zwar schon aus der GB-A-728 074 bekannt. Im Unterschied zum beanspruchten Verfahren werden sie jedoch im vorbeschriebenen Prozeß zur Umsetzung von Kohlenmonoxid und Wasserstoff in Gegenwart einer alkalisch reagierenden Stickstoffverbindung eingesetzt, die unter den Reaktionsbedingungen gasförmig ist. Daher führt die Umsetzung nicht zu wachsartigen Kohlenwasserstoffen, sondern zu Gemischen aus (unter Normalbedingungen) flüssigen Kohlenwasserstoffen, sauerstoffhaltigen organischen Verbindungen und stickstoffhaltigen organischen Verbindungen.

Als leichtreduzierbare Metalloxide setzt man mit Erfolg Kupferoxid, Silberoxid oder ein Gemisch dieser beiden Oxide ein. Daneben sind aber auch Oxide des Rutheniums und Palladiums allein oder im Gemisch untereinander oder mit einem oder mehreren der vorgenannten Oxide als Bestandteile der Katalysatoren geeignet, die im erfindungsgemäßen Verfahren eingesetzt werden.

Als schwerreduzierbares Metalloxid hat sich besonders Titandioxid bewährt. Es kann in Form des handelsüblichen $TiO_2$ eingesetzt werden, aber auch als Oxidhydrat, wie es z. B. bei der Umsetzung wasserlöslicher Ti(IV)-Verbindungen mit alkalisch reagierenden Reagenzien anfällt. Neben Titandioxid eigenen sich Vanadinpentoxid und Chrom(III)-oxid als schwerreduzierbare oxidische Bestandteile des Katalysators. Auch die schwerreduzierbaren Metalloxide können im Katalysator allein oder in Form eines Gemisches aus zwei oder mehr Bestandteilen enthalten sein.

Die Zusammensetzung der nach dem erfindungsgemäßen Verfahren verwendeten Katalysatoren kann in weiten Grenzen variieren. Ein gemeinsames Merkmal der Katalysatoren ist jedoch, daß ihr Hauptbestandteil Eisen ist. Bevorzugt setzt man Katalysatoren ein, die auf 100 Gew.-Teile Eisen 0,1 bis 10 Gew.-Teile und insbesondere 0,5 bis 6 Gew.-Teile des leichtreduzierbaren Metalloxids enthalten. Sehr bewährt hat es sich, Katalysatoren einzusetzen, die auf 100 Gew.-Teile Eisen 1 bis 25 Gew.-Teile und insbesondere 5 bis 15 Gew.-Teile des schwerreduzierbaren Metalloxids enthalten. Ferner enthält der Katalysator auf 100 Gew.-Teile Eisen 5 bis 35 und insbesondere 10 bis 30 Gew.-Teile Siliciumdioxid. Als Siliciumdioxid können die verschiedensten Formen der Verbindung $SiO_2$ eingesetzt werden, auch solche, die Wasser in gebundener Form enthalten, darüber hinaus Silikate, von denen sich Alkalisilikate besonders bewährt haben. Schließlich ist der Katalysator noch durch einen Gehalt an Alkalimetalloxid charakterisiert. Auf 100 Gew.-Teile Eisen enthält es vorzugsweise 1 bis 30 und insbesondere 1 bis 15 Gew.-Teile Alkalimetalloxid. Das Alkalimetalloxid wird nicht als solches dem Katalysator zugesetzt, sondern z. B. in Form der Hydroxide, der Karbonate oder Silikate. Bevorzugt wird der Einsatz von Natrium- und/oder Kaliumverbindungen.

Zur Herstellung der Katalysatoren kann man sich verschiedener Verfahren bedienen. Eine Arbeitsweise geht von löslichen Verbindungen des Eisens sowie der Verbindungen aus, die leichtreduzierbare bzw. schwerreduzierbare Metalloxide bilden und fällt durch Einwirkung alkalischer Reagenzien, z. B.

Alkalikarbonat oder Alkalihydroxid, die entsprechenden Karbonate oder Hydroxide. Anschließend wird die Katalysatormasse abfiltriert, gewaschen und mit Alkalisilikat imprägniert. Eine andere Arbeitsweise besteht darin, $TiO_2$ vorzulegen, die übrigen Komponenten auf diesem Feststoff aufzufällen und mit Alkalisilikat zu imprägnieren. Nach einem weiteren Verfahren legt man $TiO_2$ und $SiO_2$ gemeinsam vor und fällt die übrigen Komponenten auf das Gemisch der Oxide. Anschließend wird der Katalysator mit Alkalisilikat und/oder mit Alkalicarbonat und/oder Alkalihydroxid imprägniert. Auch die Imprägnierung von $SiO_2$ und gegebenenfalls $TiO_2$ mit wässrigen Lösungen eines Eisensalzes sowie eines Salzes des Metalls, das ein leichtreduzierbares Oxid ergibt, hat sich zur Herstellung der Katalysatoren bewährt.

Die nach einem der beschriebenen Verfahren erhaltene Katalysatormasse wird schließlich geformt, z. B. durch Extrudieren oder durch Herstellung von Bruchkorn, Tabletten oder Pellets.

Zur Reduktion wird der Katalysator mit Wasserstoff, Kohlenmonoxid oder Mischungen der beiden Gase bei Temperaturen von 150 bis 350 °C reduziert. Es hat sich bewährt, die Reduktion des Katalysators bei linearen Gasgeschwindigkeiten von 10 bis 500 cm/sec. (berechnet auf Normalbedingungen und bezogen auf den leeren Reaktor) und insbesondere bei 50 bis 250 cm/sec. durchzuführen. Hierbei wird ein Druck von 1 bis 50 bar eingehalten, vorzugsweise von 1 bis 25 bar und insbesondere von 1 bis 10 bar. Die Behandlung des Katalysators mit Kohlenmonoxid, Wasserstoff oder einem Gemisch aus Kohlenmonoxid und Wasserstoff wird so lange durchgeführt, bis 10 bis 50 Gew.-%, vorzugsweise 20 bis 35 Gew.-% metallisches Eisen, bezogen auf den gesamten Eisengehalt des Katalysators, vorhanden sind.

Die Umsetzung kann bei den für die Fischer-Tropsch-Reaktion üblichen Bedingungen hinsichtlich Temperatur, Druck, Gaszusammensetzung und Raumgeschwindigkeit durchgeführt werden, so daß man sie in den üblichen Anlagen ausüben kann. Allerdings ist zu berücksichtigen, daß durch die Einhaltung enger Druck- und Temperaturbereiche sowie bestimmter Raumgeschwindigkeiten die Selektivität der Umsetzung weiter erhöht werden kann. Unter dem Begriff « Selektivität » wird dabei der Anteil Kohlenmonoxid verstanden, der zu den gewünschten Verbindungen umgesetzt wird.

Die Umsetzung wird bei Temperaturen von 150 bis 350 °C und Drücken von 5 bis 50 bar durchgeführt. Bewährt hat es sich, Temperaturen von 180 bis 280 °C und insbesondere von 200 bis 250 °C anzuwenden. Die Drücke liegen vorzugsweise im Bereich von 20 bis 40 bar. Höhere Temperaturen führen zur vermehrten Bildung von gasförmigen und flüssigen Kohlenwasserstoffen, höhere Drücke verbessern die Selektivität bezüglich der Bildung der gewünschten Kohlenwasserstoffe. Der Anwendung höherer Drücke sind jedoch wirtschaftliche Grenzen gesetzt, die sich z. B. aus den Anforderungen an die Druckfestigkeit der Apparaturen ergeben. Das Synthesegas wird mit einer Frischgas-Raumgeschwindigkeit von 100 bis 3 000 V/Vh, vorzugsweise von 500 bis 1 500 V/Vh über den Katalysator geleitet.

Das Verhältnis von Wasserstoff zu Kohlenmonoxid im Synthesegas kann im Bereich von 1 : 0,1 bis 1 : 10, vorzugsweise 3 : 1 bis 1 : 3 variieren. Im allgemeinen führt eine Erhöhung des Wasserstoffanteils im Gasgemisch zu einer Erhöhung des Anteils niedermolekularer Kohlenwasserstoffe.

In einer speziellen Ausführungsform des erfindungsgemäßen Verfahrens setzt man dem Synthesegas bis zu 50 Vol.%, bezogen auf das Gesamtgemisch, Inertgas oder Inertgasgemisch wie Stickstoff und/oder Kohlendioxid und/oder Edelgas, zu.

Das Gas kann in geradem Durchgang über den Katalysator geführt werden. Es ist jedoch zweckmäßig, einen Teil des Gases nach Abtrennung der Reaktionsprodukte und Ergänzung von umgesetztem Wasserstoff und Kohlenmonoxid durch Frischgas im Kreis zu führen. Besonders bewährt hat es sich, das Volumverhältnis von Frischgas zu Kreislaufgas auf Werte von 1 : 1 bis 1 : 10 und insbesondere auf Werte von 1 : 1 bis 1 : 5 einzustellen.

Der Katalysator ist in Form eines Festbettes oder eines volumvergrößerten, d. h. expandierten Festbettes angeordnet. Nach einer besonderen Ausführungsform des erfindungsgemäßen Verfahrens leitet man während der Synthese kontinuierlich oder diskontinuierlich arteigenes organisches Flüssigprodukt über den Katalysator. Besonders bewährt haben sich als Flüssigprodukte Kohlenwasserstoffe mit 5 bis 20 Kohlenstoffatomen, die zu 10 bis 90 Gew.-% aus Olefinen bestehen. Die Behandlung des Katalysators mit dem arteigenen organischen Flüssigprodukt führt zu einer Erhöhung der Ausbeute an langkettigen Kohlenwasserstoffen.

Das nach dem neuen Verfahren erhaltene Syntheseprodukt besteht zu mindestens 70 Gew.-% aus Kohlenwasserstoffen mit mehr als 18 Kohlenstoffatomen. Von diesen Kohlenwasserstoffen sind mehr als 95 % linear. Gasförmige Kohlenwasserstoffe mit 2 bis 4 Kohlenstoffatomen werden in einem Anteil von weniger als 5 Gew.-% gebildet.

## Beispiel 1

Herstellung des Katalysators Nr. 1

4 078 g $Fe(NO_3)_3 \cdot 9\ H_2O$ und 107 g $Cu(NO_3) \cdot 3\ H_2O$ werden in 11 l Wasser gelöst, auf 100 °C erhitzt und innerhalb ewa 2 Min. zu einer siedenden Kaliumcarbonatlösung (11,5 Gew.-% $K_2CO_3$), die 225 ml Titanoxichloridlösung (250 g $TiO_2$/l) enthält, gegeben. Das Umsetzungsprodukf hat einen pH-Wert von 6,9. Es wird abfiltriert und mit 65 bis 75 °C warmem Wasser so lange gewaschen, bis die elektrische Leitfähigkeit des Waschwassers unter 100 µS liegt. Der Filterkuchen wird mit Wasser in Verhältnis 1 : 1 angemaischt und mit einer Kaliumsilikatlösung [150 g $K_2SiO_3$ (58 % $SiO_2$)/500 ml $H_2O$] versetzt. Man stellt mit konzentrierter Salpetersäure auf pH 6,9 ein, erhitzt auf 85 °C und filtriert erneut.

Der feuchte Filterkuchen wird bei etwa 50 °C getrocknet. Der fertige Katalysator hat folgende Zusammensetzung (in Gew.-Teilen) :

100 Fe : 4 Cu : 7 $TiO_2$ : 15 $SiO_2$ : 8 $K_2O$

Beispiel 2

Herstellung des Katalysators Nr. 2

4 078 g $Fe(NO_3)_3 \cdot 9\ H_2O$ und 107 g $Cu(NO_3)_2 \cdot 3\ H_2O$ werden in 11 l Wasser gelöst, auf 100 °C erhitzt und innerhalb etwa 2 Min. zu einer siedenden Kaliumcarbonatlösung (11,5 Gew.-% $K_2CO_3$), die 56,3 g Titanoxid enthält, gegeben. Das Umsetzungsprodukt hat einen pH-Wert von 6,9. Es wird' abfiltriert und mit 65 bis 75 °C warmem Wasser so lange gewaschen, bis die elektrische Leitfähigkeit des Waschwassers bei etwa 200 µS liegt. Der Filterkuchen wird mit Wasser im Verhältnis 1 : 1 angemaischt und mit Kaliumsilikatlösung (150 g $K_2SiO_3$ (58 % $SiO_2$)/500 ml $H_2O$) versetzt. Man stellt mit konzentrierter Salpetersäure auf pH 6,9 ein, erhitzt auf 85 °C und filtriert erneut. Anschließend wird der Filerkuchen bei etwa 50 °C getrocknet. Der fertige Katalysator hat folgende Zusammensetzung (in Gew.-Teilen) :

100 Fe : 6 Cu : 8 $TiO_2$ : 18 $SiO_2$ : 6 $K_2O$

In dem Katalysator kann Kupfer teilweise oder vollständig durch Silber ersetzt werden.

Beispiel 3

Herstellung des Katalysators Nr. 3

4 078 g $Fe(NO_3)_3 \cdot 9\ H_2O$ und 107 g $Cu(NO_3)_2 \cdot 3\ H_2O$ werden in 11 l $H_2O$ gelöst, auf 100 °C erhitzt und innerhalb etwa 2 Min. zu einer siedenden Sodalösung (11,5 Gew.-% $Na_2CO_3$), die 225 ml Titanoxichloridlösung (250 g $TiO_2$/l) enthält, gegeben. Das Umsetzungsprodukt hat einen pH-Wert von 6,9. Es wird abfiltriert und mit 65 bis 75 °C warmem Wasser so lange gewaschen, bis die elektrische Leitfähigkeit des Waschwassers bei etwa 100 µS liegt. Das Filtrat wird mit Wasser im Verhältnis 1 : 1 angemaischt und mit Kaliumsilikatlösung [250 g $K_2SiO_3$ (58 % $SiO_2$)/600 ml $H_2O$] versetzt. Man stellt mit konzentrierter Salpetersäure auf pH 6,9 ein, erhitzt auf 85 °C und filtriert erneut. Der feuchte Filterkuchen wird bei etwa 50 °C getrocknet. Der fertige Katalysator hat folgende Zusammensetzung (in Gew.-Teilen) :

100 Fe : 5 Cu : 8 $TiO_2$ : 30 $SiO_2$ : 5$K_2O$

In dem Katalysator kann Kupfer teilweise oder ganz durch Silber ersetzt werden.

Beispiel 4

Herstellung des Katalysators Nr. 4

4 078 g $Fe(NO_3)_3 \cdot 9\ H_2O$ und 107 g $Cu(NO_3)_2 \cdot 3\ H_2O$ werden in 11 l $H_2O$ gelöst, auf 100 °C erhitzt und innerhalb etwa 2 Min. zu einer siedenden Sodalösung (11,5 Gew.-% $Na_2CO_3$), die 225 ml Titanoxichloridlösung (250 g $TiO_2$/l) enthält, gegeben. Das Umsetzungsprodukt hat einen pH-Wert von 6,9. Es wird abfiltriert und mit 65 bis 75 °C warmem Wasser so lange gewaschen, bis die elektrische Leitfähigkeit des Waschwassers bei etwa 150 µS liegt. Der Filterkuchen wird mit Wasser im Verhältnis 1 : 1 angemaischt und mit Kaliumsilikatlösung [150 g $K_2SiO_3$ (58 % $SiO_2$)/500 ml $H_2O$] versetzt. Man stellt mit konzentrierter Salpetersäure auf pH 6,9 ein, erhitzt auf 85 °C und filtriert erneut. Der feuchte Filterkuchen wird bei etwa 50 °C getrocknet. Der fertige Katalysator hat folgende Zusammensetzung (in Gew.-Teilen) :

100 Fe : 5 Cu : 9 $TiO_2$ : 15 $SiO_2$ : 3 $K_2O$

Reduktion der Katalysatoren und Synthese

Die Reduktion der nach den Beispielen 1 bis 4 hergestellten Katalysatoren erfolgt in gleicher Weise mit Wasserstoff, der von oben nach unten über den Katalysator geleitet wird. Das Aufheizen bis zum Erreichen der Reduktionstemperatur wird in $H_2$-Atmosphäre durchgeführt. Die Reduktion erfolgt unter den nachstehenden Bedingungen :

| | |
|---|---|
| Dauer : | 1 h |
| Temperatur (°C) : | 240 °C |
| $H_2$-Gasgeschwindigkeit : | 150 cm/sec = 650 V/Vh |

Nach Beendigung der Reduktion wird auf etwa 160 bis 170 °C abgekühlt und Wasserstoff bis 20 bar Druck zugeführt. Anschließend wird der Gaskreislauf eingestellt, mit der CO $H_2$-Dosierung begonnen, und der Katalysator sehr langsam auf Synthesetemperatur gebracht. Die Syntheseergebnisse sind in der folgenden Tabelle aufgeführt.

Tabelle

| Katalysator | | 1 | 2 | 3 | 4 |
|---|---|---|---|---|---|
| Temperatur (°C ; gemessen im Katalysator) | | 220 | 207 | 208 | 195 |
| Druck (bar) | | 20 | 20 | 20 | 21,4 |
| Raumgeschwindigkeit (V/Vh) | | 498 | 494 | 441 | 494 |
| $X_{H_2}/CO$ | | 1,05 | 1,01 | 1,57 | 1,88 |
| $(CO + H_2)$-Umsatz | (%) | 26,6 | 29,0 | 16,8 | 20,0 |
| Wachs-Selektivität bez. auf CO-Umsatz | (%) | 89,3 | 75,1 | 70,6 | 76,9 |
| Ausbeute KW $\geqslant C_2$ | (g/Nm³) | 52,8 | 57,8 | 31,0 | 38,1 |
| Wachs (KW $\geqslant C_{18}$) | (g/Nm³) | 39,4 | 39,2 | 22,7 | 31,7 |
| Benzin-Diesel | (g/Nm³) | 10,1 | 9,8 | 4,0 | 6,2 |
| Methan | (g/Nm³) | 1,5 | 1,4 | 2,8 | 2,0 |
| $C_2$-$C_7$-KW | (g/Nm³) | 3,3 | 8,8 | 4,3 | 0,2 |

**Patentansprüche**

1. Verfahren zur Herstellung von linearen Kohlenwasserstoffen mit mehr als 18 Kohlenstoffatomen durch Umsetzung von Kohlenmonoxid und Wasserstoff enthaltenden Gasgemischen an Eisen enthaltenden Katalysatoren bei 150 bis 350 °C und 5 bis 50 bar, dadurch gekennzeichnet, daß die Katalysatoren zusätzlich ein leichtreduzierbares Metalloxid, ein schwerreduzierbares Metalloxid, $SiO_2$ und Alkalimetalloxid enthalten.

2. Verfahren nach Anspruch 1, dadurch gekennzeichnet, daß das leichtreduzierbare Metalloxid Kupferoxid und/oder Silberoxid ist.

3. Verfahren nach Anspruch 1, dadurch gekennzeichnet, daß das schwerreduzierbare Metalloxid Titandioxid ist.

4. Verfahren nach Anspruch 1 und 2, dadruch gekennzeichnet, daß der Katalysator auf 100 Gew.-Teile Eisen 0,1 bis 10, vorzugsweise 0,5 bis 6 Gew.-Teile des leichtreduzierbaren Metalloxids enthält.

5. Verfahren nach Anspruch 1 und 3, dadurch gekennzeichnet, daß der Katalysator auf 100 Gew.-Teile Eisen 1 bis 25, vorzugsweise 5 bis 15 Gew.-Teile des schwerreduzierbaren Metalloxids enthält.

6. Verfahren nach Anspruch 1 bis 5, dadurch gekennzeichnet, daß der Katalysator auf 100 Gew.-Teile Eisen 5 bis 35, vorzugsweise 10 bis 30 Gew.-Teile $SiO_2$ enthält.

7. Verfahren nach Anspruch 1 bis 6, dadurch gekennzeichnet, daß der Katalysator auf 100 Gew.-Teile Eisen 1 bis 30, vorzugsweise 1 bis 15 Gew.-Teile Alkalimetalloxid enthält.

**Claims**

1. Process for producing linear hydrocarbons with more than 18 carbon atoms by converting gas mixtures containing carbon monoxide and hydrogen with iron-containing catalysts at 150 to 350 °C and 5 to 50 bars, characterised in that the catalysts in addition contain a readily reducible metal oxide, a difficultly reducible metal oxide, $SiO_2$, and an alkali metal oxide.

2. Process according to claim 1, characterised in that the readily reducible metal oxide is cupric oxide and/or silver oxide.

3. Process according to claim 1, characterised in that the difficultly reducible metal oxide is titanium dioxide.

4. Process according to claims 1 and 2, characterised in that the catalyst contains 0.1 to 10, preferably 0.5 to 6 parts by weight of the readily reducible metal oxide per 100 parts by weight of iron.

5. Process according to claims 1 and 3, characterised in that the catalyst contains 1 to 25, preferably 5 to 15 parts by weight of the difficultly reducible metal oxide per 100 parts by weight of iron.

6. Process according to claims 1 to 5, characterised in that the catalyst contains 5 to 35, preferably 10 to 30 parts by weight of $SiO_2$ per 100 parts by weight of iron.

7. Process according to claims 1 to 6, characterised in that the catalyst contains 1 to 30, preferably 1 to 15 parts by weight of alkali metal oxide per 100 parts by weight of iron.

**Revendications**

1. Procédé pour la fabrication d'hydrocarbures linéaires en plus de $C_{18}$ par réaction de mélanges aqueux contenant du monoxyde de carbone et de l'hydrogène sur des catalyseurs contenant du fer à 150-350 °C et sous 5-50 bars, caractérisé en ce que les catalyseurs contiennent en outre un oxyde métallique facilement réductible, un oxyde métallique difficilement réductible, $SiO_2$ et un oxyde de métal alcalin.

2. Procédé selon la revendication 1, caractérisé en ce que l'oxyde métallique facilement réductible est l'oxyde de cuivre et/ou l'oxyde d'argent.

3. Procédé selon la revendication 1, caractérisé en ce que l'oxyde métallique difficilement réductible est le dioxyde de titane.

4. Procédé selon les revendications 1 et 2, caractérisé en ce que le catalyseur contient 0,1 à 10 parties en poids, en particulier 0,5 à 6 parties en poids, de l'oxyde métallique facilement réductible, pour 100 parties en poids de fer.

5. Procédé selon les revendications 1 et 2, caractérisé en ce que le catalyseur contient 1 à 25 parties en poids, en particulier 5 à 15 parties en poids, de l'oxyde métallique difficilement réductible, pour 100 parties en poids de fer.

6. Procédé selon les revendications 1 à 5, caractérisé en ce que le catalyseur contient en outre 5 à 35 parties en poids, en particulier 10 à 30 parties en poids, de dioxyde de silicium pour 100 parties en poids de fer.

7. Procédé selon les revendications 1 à 6, caractérisé en ce que le catalyseur contient 1 à 30 parties en poids, de préférence 1 à 15 parties en poids, d'oxyde de métal alcalin pour 100 parties en poids de fer.